(19) Europäisches Patentamt
European Patent Office
Office européen des brevets

(11) Veröffentlichungsnummer: **0 367 069 B1**

(12) # EUROPÄISCHE PATENTSCHRIFT

(45) Veröffentlichungstag der Patentschrift: **19.01.94**

(51) Int. Cl.5: **C07D 403/08**, C07D 249/10, C07D 405/08, C07D 403/06, A61K 31/41

(21) Anmeldenummer: **89119693.3**

(22) Anmeldetag: **24.10.89**

Die Akte enthält technische Angaben, die nach dem Eingang der Anmeldung eingereicht wurden und die nicht in dieser Patentschrift enthalten sind.

(54) **Substituierte Bisazole, Verfahren zu ihrer Herstellung und ihre Verwendung als Arzneimittel.**

(30) Priorität: **04.11.88 DE 3837463**

(43) Veröffentlichungstag der Anmeldung:
**09.05.90 Patentblatt 90/19**

(45) Bekanntmachung des Hinweises auf die Patenterteilung:
**19.01.94 Patentblatt 94/03**

(84) Benannte Vertragsstaaten:
**AT BE CH DE ES FR GB GR IT LI LU NL SE**

(56) Entgegenhaltungen:
**EP-A- 0 122 693**
**EP-A- 0 164 246**
**EP-A- 0 180 835**
**EP-A- 0 180 838**

(73) Patentinhaber: **BAYER AG**

**D-51368 Leverkusen(DE)**

(72) Erfinder: **Stroech, Klaus, Dr.**
**Rolsberger Strasse 22**
**D-5650 Solingen 19(DE)**
Erfinder: **Backens-Hammerschmidt, Susanne, Dr.**
**Kicke 19**
**D-5060 Bergisch-Gladbach 1(DE)**
Erfinder: **Plempel, Manfred, Dr.**
**Zwengenbergerstrasse 3c**
**D-5657 Haan(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 367 069 B1

## Beschreibung

Die vorliegende Erfindung betrifft neue substituierte 1-[1-(3-Chlor-1,2,4-triazol-1-yl)-cyclopropyl]-2-(1,2,4-triazol-1-yl)-ethan-1-ol-Derivate, Verfahren zu ihrer Herstellung und ihre Verwendung bei der Herstellung von Arzneimittel, insbesondere von Antimykotika.

Es ist bereits bekannt, daß bestimmte Diazolyl-Derivate und substituierte Azolylcyclopropyl-azolylmethyl-carbinol-Derivate gute antimikrobielle, insbesondere antimykotische Eigenschaften aufweisen [vgl. EP-OS 0 044 605 und DE 34 40 114 A1]. Außerdem sind aus EP-A-018035, EP-A-018038 und EP-A-0164246 Azolyl-cyclopropyl-azolylmethyl carbinole bekannt, die jedoch keine Halogen substitution an der Azolyl-cyclopropyl-Gruppe Tragen. Die Wirkung dieser Stoffe ist jedoch nicht immer in allen Indikationsbereichen voll zufriedenstellend.

Es wurden nun neue substituierte Azolyl-cyclopropyl-azolylmethyl-carbinole der allgemeinen Formel (I)

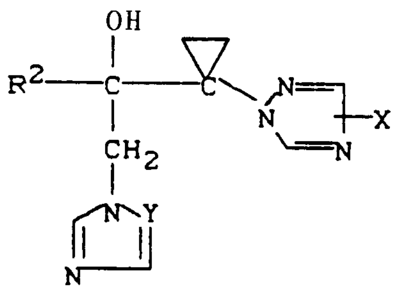

(I)

in welcher

$R^2$ - für Aryl mit 6 bis 10 Kohlenstoffatomen steht, das gegebenenfalls bis zu 4-fach gleich oder verschieden substituiert ist durch Halogen, $C_1$-$C_8$-Alkyl, $C_1$-$C_8$-Alkoxy, $C_1$-$C_8$-Alkylthio, oder durch Halogenalkyl, Halogenalkoxy oder Halogenalkylthio mit bis zu 8 Kohlenstoffatomen im Alkylteil, wobei die Halogenatome gleich oder verschieden sind oder durch Phenyl, Phenylthio oder Phenoxy, welches seinerseits bis zu 3-fach gleich oder verschieden durch Halogen substituiert sein kann, oder

X - für Halogen steht,

und

Y - für ein Stickstoffatom oder für die CH-Gruppe steht

und deren Säureadditionssalze gefunden.

Salze der erfindungsgemäßen Verbindungen sind Additionsprodukte mit Säuren. Zu den Säuren, die addiert werden können, gehören vorzugsweise physiologisch verträgliche Säuren, insbesondere Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bisfunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Bevorzugt sind Verbindungen der allgemeinen Formel (I),

in welcher

$R^2$ - für Phenyl oder Naphthyl steht, das gegebenenfalls bis zu 3-fach gleich oder verschieden substituiert ist durch Halogen, $C_1$-$C_6$-Alkyl, $C_1$-$C_6$-Alkoxy, $C_1$-$C_6$-Alkylthio oder durch Halogenalkyl, Halogenalkoxy oder Halogenalkylthio mit bis zu 6 Kohlenstoffatomen im Alkylteil, wobei die Halogenatome gleich oder verschieden sind, oder durch Phenyl, Phenylthio, oder Phenoxy, welches seinerseits bis zu 2-fach gleich oder verschieden durch Fluor, Chlor oder Brom substituiert sein kann, oder

X - für Halogen steht,

und

Y - für ein Stickstoffatom oder für die CH-Gruppe steht

und deren Säureadditionssalze.

Besonders bevorzugt sind Verbindungen der allgemeinen Formel (I),

in welcher

$R^2$ - für Phenyl oder Naphthyl steht, das gegebenenfalls bis zu 2-fach gleich oder verschieden substituiert ist durch Fluor, Chlor, Brom, $C_1$-$C_4$-Alkyl, $C_1$-$C_4$-Alkoxy, $C_1$-$C_4$-Alkylthio oder durch

Halogenalkyl, Halogenalkoxy oder Halogenalkylthio mit bis zu 4 Kohlenstoffatomen im Alkylteil, wobei die Halogenatome gleich oder verschieden sein können, oder durch Phenyl, Phenoxy oder Phenylthio, das seinerseits durch Fluor, Chlor oder Brom substituiert sein kann,

X - für Fluor, Chlor oder Brom steht

und

Y - für ein Stickstoffatom oder für die CH-Gruppe steht,

und deren Säureadditionssalze.

Die erfindungsgemäßen Stoffe enthalten ein asymmetrisches substituiertes Kohlenstoffatom. Sie können daher in optischen Isomerenformen anfallen. Die vorliegende Erfindung betrifft sowohl die einzelnen Isomere als auch deren Gemisch.

Die erfindungsgemäßen Verbindungen der allgemeinen Formel (I) und deren Säureadditionssalze zeigen gute antimikrobielle, insbesondere antimykotische Eigenschaften.

Die erfindungsgemäßen Verbindungen der allgemeinen Formel (I),

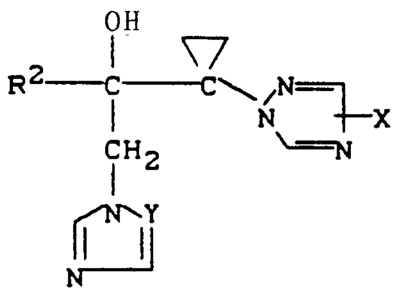

(I)

in welcher

$R^2$, X und Y die oben angegebene Bedeutung haben,

können nach bekannten Methoden hergestellt werden,

indem man

[A] halogenierte Azolylcyclopropylketone der allgemeinen Formel (II)

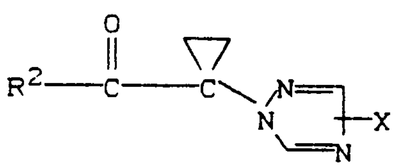

(II)

in welcher

$R^2$ und X die oben angegebene Bedeutung haben,

zunächst mit Dimethylsulfoniummethylid oder Dimethyloxosulfoniummethylid der Formel (III)

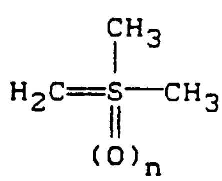

(III)

worin

n - eine Zahl 0 oder 1 bedeutet,

in Gegenwart eines Verdünnungsmittels, wie beispielsweise Dimethylsulfoxid, bei Temperaturen zwischen +20°C und +80°C [vgl. hierzu J. Am. Chem. Soc. 87, 1363 - 1364 (1965)] zu Verbindungen der allgemeinen Formel (IV)

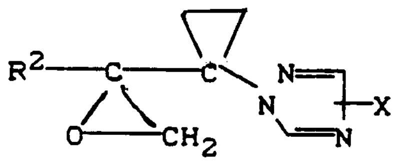

(IV)

in welcher

$R^2$ und X die oben angegebene Bedeutung haben,

umsetzt

und anschließend

mit Azolen der allgemeinen Formel (V)

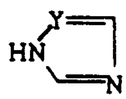

(V)

in welcher

Y die oben angegebene Bedeutung hat,

in Gegenwart eines inerten organischen Lösemittels, wie beispielsweise Acetonitril oder Dimethylformamid, in Gegenwart einer Base, wie beispielsweise Kaliumcarbonat oder Kaliumhydroxid, bei Temperaturen zwischen +40°C und +150°C zu Verbindungen der allgemeinen Formel (Ia)

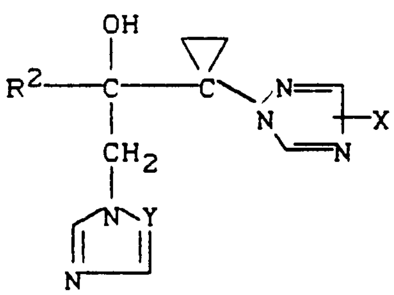

(Ia)

in welcher

$R^2$, X und Y die oben angegebene Bedeutung haben,

umsetzt,

oder indem man

[B] Diazolyl-Keton-Derivate der allgemeinen Formel (VII)

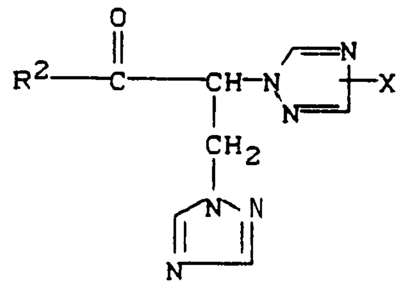

(VII)

in welcher

$R^2$ und X die oben angegebene Bedeutung haben,

zunächst mit mindestens der dreifachen äquivalenten Menge Dimethyloxosulfoniummethylid der Formel (IIIa)

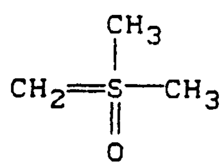

(IIIa)

nach der unter Variante [A] angegebenen Methode umsetzt.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösemittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösemittel gereinigt werden.

Die Aryl-azolylcyclopropylketone der allgemeinen Formel (II) sind neu. Sie können erhalten werden, indem man

Aryl-halogenpropylketone der allgemeinen Formel (VIII)

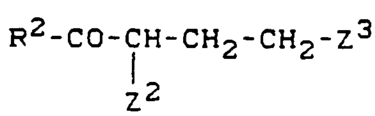

(VIII)

in welcher

$R^2$ die oben angegebene Bedeutung hat

und

$Z^2$ und $Z^3$ gleich oder verschieden sind und vorzugsweise für Brom oder Chlor stehen,

mit Azolen der allgemeinen Formel (IX)

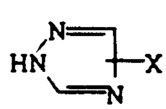

(IX)

in welcher

X die oben angegebene Bedeutung hat,

in Gegenwart eines inerten organischen Lösemittels, wie beispielsweise Acetonitril oder Dimethylformamid, in Gegenwart einer Base, wie beispielsweise Kaliumcarbonat oder Kaliumhydroxid, bei Temperaturen zwischen +40 °C und +150 °C umsetzt.

Das als Ausgangsstoff eingesetzte Dimethyloxosulfoniummethylid der Formel (III) wird in situ durch Umsetzung von Trimethyloxosulfoniumjodid mit Natriumhydrid oder Natriumamid, insbesondere mit Kalium-tert.butylat oder Natriumethylat, in Gegenwart eines Verdünnungsmittels erzeugt.

Die Azole der allgemeinen Formel (V) und (XI) sind bekannt [vgl. K. Schofield, M.R. Grimmett, B.R.T. Keene, "The Azoles", Cambrigde University Press, Cambridge 1976].

Die Verbindungen der allgeinem Formel (VIII) sind bekannt oder können nach bekannten Methoden hergestellt werden [vgl. DE-OS 25 21 104, DE-OS 23 20 355 und DE-OS 23 51 948].

Die Diazolyl-Keto-Derivate der allgemeinen Formel (VII) sind neu und können hergestellt werden, indem man

Verbindungen der allgemeinen Formel (X)

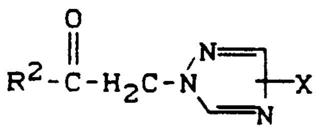

(X)

in welcher

$R^2$ und X die oben angegebene Bedeutung haben,

mit Hydroxymethylazolen der allgemeinen Formel (XI)

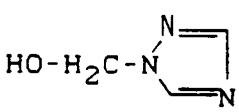

(XI)

in Gegenwart eines inerten organischen Verdünnungsmittels, wie beispielsweise Toluol, und in Gegenwart eines Katalysators, wie beispielsweise Piperidinacetat, vorzugsweise bei der Siedetemperatur des verwendeten Lösemittels umsetzt.

Die Verbindungen der allgemeinen Formel (X) können in allgemein üblicher Weise hergestellt werden [vgl. DE-OS 24 31 407; DE-OS 26 10 022 und DE-OS 26 38 470].

Ebenso ist die Verbindung der Formel (XI) bekannt [vgl. EP 0 006 102 und Chem. Heterocycl. Comp. 1980, 189].

Die erfindungsgemäß verwendbaren Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus,Trichphyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:

Dermatomykosen und Systemmykosen durch Trichophyton mentagrophytes und andere Trichophytonarten, Microsporonarten, Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht-toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen, sowie Verfahren zur Herstellung

dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenen Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Olivenöl, Ricinusöl und Sesamöl, Glycerin, Glycerinformal, Tetrahydofurfurylalkohol, Polyethylenglykole und Fettsäureester des Sorbitan oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oraler Applikation werden die erfindungsgemäßen Wirkstoffe in Gesamtmengen von etwa 2,5 bis etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den obengenannten Dosierungen abzuweichen und zwar in Abhängigkeit von der Art und dem Körpergewicht des zu behandelnden Objekts, der Art der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben aufgeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Ausgangsverbindungen und Herstellungsbeispiele

Beispiel 1

1-(4-Chlorbenzoyl)-1-(3-chlor-1,2,4-triazol-1-yl)-cyclopropan

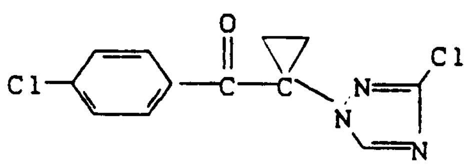

34 g (246 mmol) Kaliumcarbonat und 35 g (338 mmol) 3-Chlor-1,2,4-triazol werden in 130 ml Aceton unter Rückfluß vorgelegt und 50 g (169 mol) 2-Brom-4-chlor-1-(4-chlorphenyl)-butan-1-on in 60 ml Aceton zugetropft. Man kocht 8 Stunden unter Rückfluß, saugt vom Rückstand ab und zieht das Lösemittel im Vakuum ab. Es wird in Essigsäureethylester aufgenommen, mit Wasser gewaschen, über Natriumsulfat getrocknet und das Lösemittel abgedampft. Chromatographie an Kieselgel (Laufmittel: Dichlormethan) ergibt 42,9 g der Titelverbindung (90% der Theorie) mit dem Fp. 85 °C.

Beispiel 2

1-(3-Chlor-1,2,4-triazol-1-yl)-1-(4-fluorbenzoyl)-cyclopropan

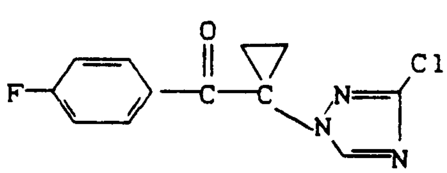

Die Verbindung aus Beispiel 2 wurde in Analogie zu der Vorschrift von Beispiel 1 hergestellt.
Fp.: 84 °C

Beispiel 3

2-(4-Chlorphenyl)-2-[1-(3-chlor-1,2,4-triazol-1-yl)-cyclopropyl]-oxiran

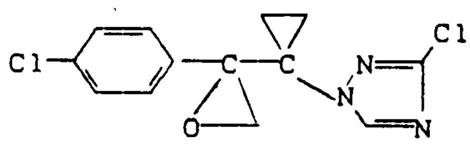

Unter Stickstoffatmosphäre werden 2,5 g (83 mmol) Natriumhydrid (80%ig) und 17,6 g (80 mmol) Trimethylsulfoxoniumiodid vorgelegt und bei 10 °C 60 ml absolutes Dimethylsulfoxid zugetropft. Man rührt 1 Stunde bei 20 °C nach und gibt anschließend bei 10 °C 20 g (71 mmol) 1-(4-Chlorbenzoyl)-1-(3-chlor-1,2,4-triazol-1-yl)-cyclopropan gelöst in 30 ml absolutem Dimethylsulfoxid zu. Nach 48 Stunden bei 20 °C wird 1 Stunde auf 40 °C erwärmt und anschließend die Reaktionslösung auf Wasser gegossen. Man extrahiert mit Essigsäureethylester, wäscht die vereinigten organischen Phasen mit Wasser, trocknet über Natriumsulfat und dampft das Lösemittel im Vakuum ab. Es bleiben 21 g der Titelverbindung (100% der Theorie) in Form eines Öls.

$^1$H-NMR (200 MHz, $CDCl_3$): δ = 0,8 - 1,4 (m, 4H); 2,92 (d, 1H); 3,15 (d, 1H); 7,10 (d, 2H); 7,38 (d, 2H); 7,75 (s, 1H).

Beispiel 4

1-(4-Chlorphenyl)-1-[1-(3-chlor-1,2,4-triazol-1-yl)-cyclopropyl]-2-(1,2,4-triazol-1-yl)-ethan-1-ol

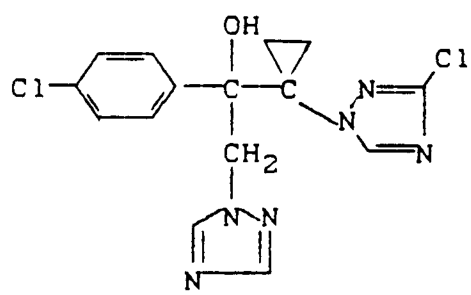

15,6 g (0,276 mol) 1,2,4-Triazol und 1,7 g (0,015 mol) Kalium-tert.butylat werden in 40 ml abs. Dimethylformamid vorgelegt und bei 80 °C 21 g (0,071 mol) 2-(4-Chlorphenyl)-2-[1-(3-chlor-1,2,4-triazol-1-yl)-cyclopropyl]-oxiran in 30 ml absolutem Dimethylformamid zugetropft. Man läßt 6 Stunden bei 100 °C reagieren und dampft hiernach das Lösemittel im Vakuum ab. Der Rückstand wird in Essigsäureethylester/Toluol aufgenommen, mit Wasser gewaschen, über Natriumsulfat getrocknet und das Lösemittel im Vakuum abgezogen. Chromatographie an Kieselgel (Laufmittel: Dichlormethan/Ethanol = 98/2) liefert 11,5 g der Titelverbindung mit dem Fp. 156 °C.

Beispiel 5

1-(4-Chlorphenyl)-1-[1-(3-chlor-1,2,4-triazol-1-yl)-cyclopropyl]-2-(imidazol-1-yl)-ethan-1-ol

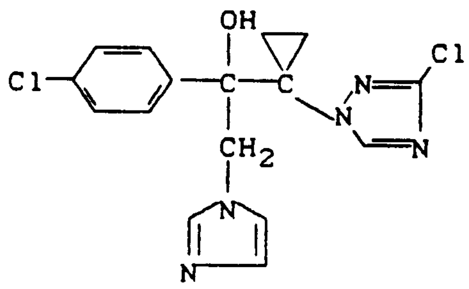

Unter Stickstoffatmosphäre werden 13 g (191 mmol) Imidazol und 1 g (9 mmol) Kalium-tert.butylat in 100 ml Acetonitril unter Rückfluß vorgelegt und 18 g (61 mmol) 2-(4-Chlorphenyl)-2-[1-(3-chlor-1,2,4-triazol-1-yl)-cyclopropyl]-oxiran gelöst in 30 ml Acetonitril zugetropft. Man läßt 10 Stunden unter Rückflußbedingungen reagieren und dampft anschließend das Lösemittel im Vakuum ab. Der Rückstand wird in Essigsäureethylester aufgenommen und mit Wasser gewaschen Man trocknet über Natriumsulfat und entfernt im Vakuum das Lösemittel. Chromatographische Reinigung an Kieselgel (Lösemittel: Dichlormethan/Ethanol = 90/10) ergibt 7,1 g (32% der Theorie) der Titelverbindung mit dem Fp. 231 °C.

Die folgenden Beispiele wurden analog der Vorschrift von Beispiel 4 hergestellt:

Beispiel 6

1-[1-(3-Chlor-1,2,4-triazol-1-yl)-cyclopropyl]-1-phenyl-2-(1,2,4-triazol-1-yl)-ethan-1-ol

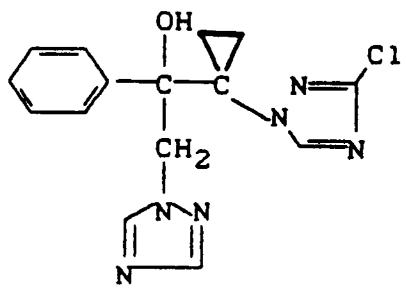

Fp.: 134 °C

Beispiel 7

1-[1-(3-Chlor-1,2,4-triazol-1-yl)-cyclopropyl]-1-(4-fluorphenyl)-2-(1,2,4-triazol-1-yl)-ethan-1-ol

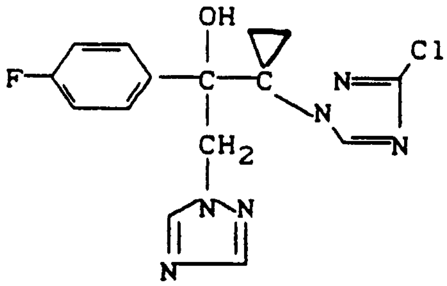

Fp.: 116 ° C

Beispiel 8

1-[1-(3-Chlor-1,2,4-triazol-1-yl)-cyclopropyl]-1-(4-methylphenyl)-2-(1,2,4-triazol-1-yl)-ethan-1-ol

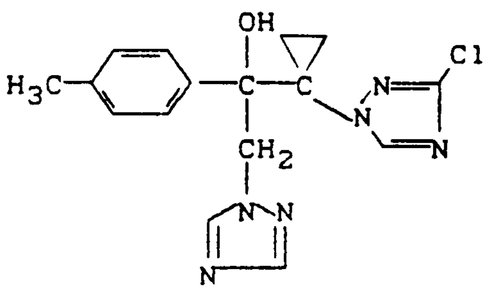

Fp.: 119 ° C

Beispiel 9

1-(4-Biphenyl)-1-[1-(3-chlor-1,2,4-triazol-1-yl)-cyclopropyl]-2-(1,2,4-triazol-1-yl)-ethan-1-ol

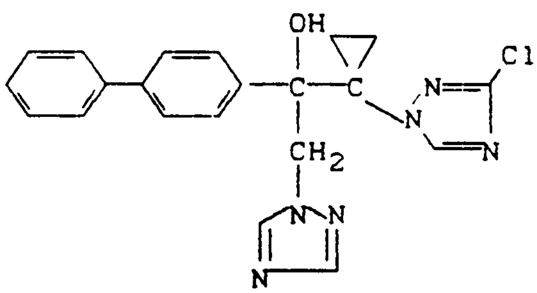

Fp.: 150 ° C

Beispiel 10

1-[1-(3-Chlor-1,2,4-triazol-1-yl)-cyclopropyl]-1-(2,4-difluorphenyl)-2-(1,2,4-triazol-1-yl)-ethan-1-ol

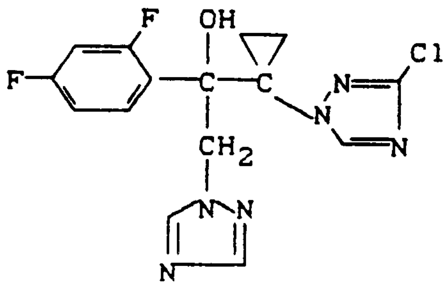

Fp.: 114 ° C

Beispiel 11

1-[1-(3-Chlor-1,2,4-triazol-1-yl)-cyclopropyl]-1-(3,4-difluorphenyl)-2-(1,2,4-triazol-1-yl)-ethan-1-ol

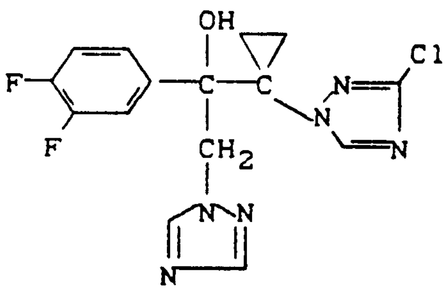

Fp.: 106 ° C

Verwendungsbeispiele

In dem in-vitro-Test werden die nachstehend angegebenen, aus der EP-OS 0 044 605 (A, B) und EP-OS 0 180 850 (C und D) bekannten Verbindungen als Vergleichssubstanzen eingesetzt:

(A) 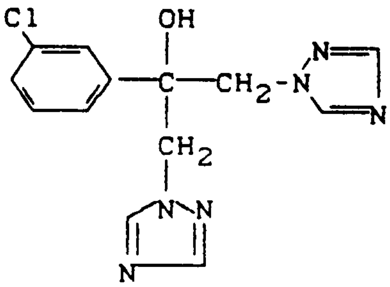

(B) 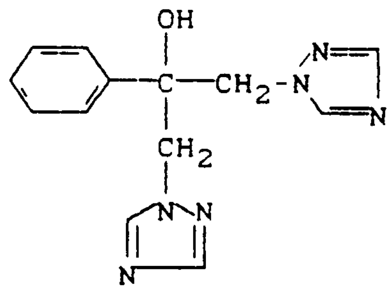

(C) 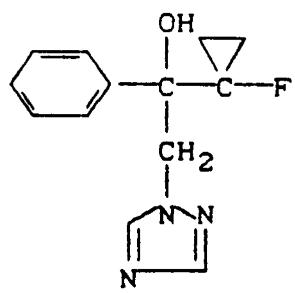

(D) 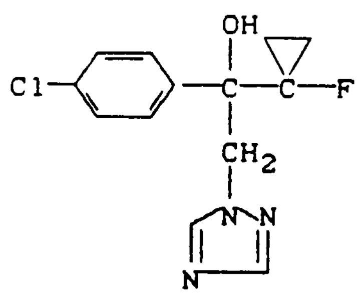

Beispiel A

Antimykotische in-vitro-Wirksamkeit

Versuchsbeschreibung:

Die in-vitro-Prüfungen wurden im Reihenverdünnungstest mit Keiminokula von durchschnittlich 5 x $10^3$ bis $10^4$ Keimen/ml Substrat durchgeführt. Als Nährmedium dienten

a) für Dermatophyten und Schimmelpilze: Kimmig's milieu d'epreuve

b) für Hefen: Fleischextrakt-Traubenzucker-Bouillon.

Die Bebrütungstemperatur betrug 28 bis 37°C, die Bebrütungsdauer lag bei 24 bis 48 Stunden bei Hefen und 96 Stunden bei Dermatophyten und Schimmelpilzen.

In diesem Test zeigten z.B. die erfindungsgemäßen Verbindungen 4, 6, 7, 8, 9, 10 und 11 eine bessere antimykotische Wirkung als die aus dem Stand der Technik bekannten Verbindungen (A) und (B).

Tabelle A

Antimykotische in-vitro-Wirksamkeit

| | MHK-Werte in µg/ml Nährmedium | | | |
|---|---|---|---|---|
| Wirkstoff | Trichophyton mentagr. | Candida albicans | Torulopsis glabrata | Aspergillus fumigatus |
| (A) bek. | 32 | >64 | >64 | >64 |
| (B) bek. | 64 | 64 | >64 | >64 |
| Verbindungen gemäß Herstellungsbeispiel | | | | |
| 4 | 4 | 8 | >64 | 32 |
| 6 | 8 | 32 | >64 | 16 |
| 7 | 8 | 32 | >64 | 32 |
| 8 | 4 | 16 | >64 | 16 |
| 9 | 4 | 32 | 16 | 16 |
| 10 | 16 | 32 | >64 | 16 |
| 11 | 16 | 16 | >64 | 64 |

Beispiel B

Antimykotische in-vivo-Wirksamkeit (oral) bei Mäusecandidose

Versuchsbeschreibung:

Mäuse vom Typ SPF-$CF_1$ wurden intravenös mit 1-2 x $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 10 bis 100 mg/kg Körper gewicht der Präparate oral behandelt.

Ergebnis

Unbehandelte Tiere starben am 3. bis 6. Tag post infektionem. Die Überlebensrate am 6. Tag post infektionem betrug bei unbehandelten Kontrolltieren etwa 5%.

In diesem Test zeigen z.B. die erfindungsgemäßen Verbindungen 4, 6, 7, 10 und 11 gute bit sehr gute Wirkung, d.h. > 80% Überlebende am 6. Tag p.i.

Tabelle B

Antimykotische in-vivo-Wirkung (oral) bei Mäusecandidose

| Wirkstoff | Wirkung |
|---|---|
| (C) bekannt | k.W. |
| (D) bekannt | k.W. |
| Verbindungsgemäße Herstellungsbeispiele | |
| 4 | +++++ |
| 6 | +++++ |
| 7 | +++++ |
| 10 | +++++ |
| 11 | +++++ |

Zeichenerklärung

| | | | |
|---|---|---|---|
| +++++ | = sehr gute Wirkung | = | 90% Überlebende am 6.Tag p.i. |
| ++++ | = gute Wirkung | = | 80% Überlebende am 6.Tag p.i. |
| +++ | = Wirkung | = | 60% Überlebende am 6.Tag p.i. |
| ++ | = schwache Wirkung | = | 40% Überlebende am 6.Tag p.i. |
| + | = Spur Wirkung | = | unter 40% Überlebendem am 6.Tag p.i. |
| k.W. | | = | kein Unterschied zur unbehandelten Infektionskontrolle. |

Beispiel C

Antimikrobielle in-vivo-Wirksamkeit (lokal) am Modell der experimentellen Meerschweinchen-Trichophytie

Versuchsbeschreibung:

Weiße Meerschweinchen der Rasse Pirbright-white werden auf dem geschorenen, nicht skarifizierten Rücken mit einer Mikro- und Makrokonidien-Suspension von Trichophyton mentagrophytes infiziert.

Die infizierten Tiere wurden beginnend mit dem 3. Tag p.i. 1 x täglich mit einer 0,1%igen Lösung der erfindungsgemäßen Präparate (in Dimethylsulfoxid : Glycerin = 1:4) lokal behandelt.

Ergebnis:

Bei unbehandelten Tieren entwickelte sich innerhalb von 12 Tagen p.i. das typische Bild einer Dermatophytose mit Rötung, Schuppung und Haarausfall bis zum totalen Integument-Defekt an der Infektionsstelle.

In diesem Test zeigen z.B. die erfindungsgemäßen Verbindungen 6, 10 und 11 eine sehr gute Wirkung.

Tabelle C

Antimikrobielle in-vivo-Wirksamkeit (lokal) am Modell der experimentellen Meerschweinchen-Trichophytie

| Wirkstoff | Wirkung |
|---|---|
| Verbindung gemäß Herstellungsbeispiel: | |
| 6 | +++++ |
| 10 | +++++ |
| 11 | +++++ |

Erläuterung:

| | | |
|---|---|---|
| +++++ | = sehr gute Wirkung | = keine Infektionszeichen am 12. bis 15. Tag p.i. |
| ++++ | = gute Wirkung | = geringe Rötung, vereinzelt Schuppen |
| +++ | = Wirkung | = Rötung, Schuppen ohne Haarausfall |
| ++ | = schwach Wirkung | = Rötung, Schuppen, Haarausfall |
| + | = Spur Wirkung | = flächiger Haarausfall, entzündliche Hautreaktion |

Beispiel D / Formulierungen

| 1) Lösung: | |
|---|---|
| Wirkstoff gemäß Formel (I) | 10 g |
| Alkohol, rein (96%ig) | 300 g |
| Isopropylmyristat | 526 g |
| | 836 g |

| 2) Creme: | |
|---|---|
| Wirkstoff gemäß Formel (I) | 10 g |
| Arlacel 60 (Sorbitan-monostearat) | 20 g |
| Tween 60 (Polyoxyethylen (2) -sorbitan-monostearat) | 15 g |
| Walrat, künstlich (Mischung von Estern von gesättigten Fettsäuren $C_{14}$-$C_{18}$ und Fettalkoholen $C_{14}$-$C_{18}$) | 30 g |
| Lanette O | 100 g |
| Eutanol G (2-Octyl-dodecanol) | 135 g |
| Benzylalkohol | 10 g |
| Wasser, entmineralisiert | 680 g |
| | 1000 g |

**Patentansprüche**

**1.** Azolyl-cyclopropyl-azolylmethyl-carbinole der Formel

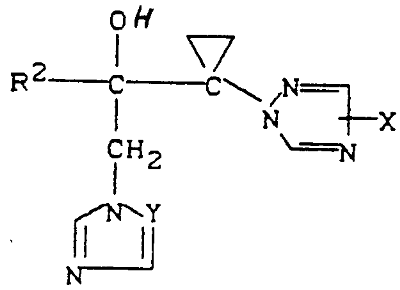

(I)

in welcher

$R^2$ - für Aryl mit 6 bis 10 Kohlenstoffatomen steht, das gegebenenfalls bis zu 4-fach gleich oder verschieden substituiert ist durch Halogen, $C_1$-$C_8$-Alkyl, $C_1$-$C_8$-Alkoxy, $C_1$-$C_8$-Alkylthio, oder durch Halogenalkyl, Halogenalkoxy oder Halogenalkylthio mit bis zu 8 Kohlenstoffatomen im Alkylteil, wobei die Halogenatome gleich oder verschieden sind oder durch Phenyl, Phenylthio oder Phenoxy, welches seinerseits bis zu 3-fach gleich oder verschieden durch Halogen substituiert sein kann,

X - für Halogen steht,

und

Y - für ein Stickstoffatom oder für die CH-Gruppe steht

und deren Säureadditionssalze.

**2.** Azolyl-cyclopropyl-azolylmethyl-carbinole gemäß Anspruch 1

in welchen

$R^2$ - für Phenyl oder Naphthyl steht, das gegebenenfalls bis zu 3-fach gleich oder verschieden substituiert ist durch Halogen, $C_1$-$C_6$-Alkyl, $C_1$-$C_6$-Alkoxy, $C_1$-$C_6$-Alkylthio oder durch Haloge-

nalkyl, Halogenalkoxy oder Halogenalkylthio mit bis zu 6 Kohlenstoffatomen im Alkylteil, wobei die Halogenatome gleich oder verschieden sind, oder durch Phenyl, Phenylthio, oder Phenoxy, welches seinerseits bis zu 2-fach gleich oder verschieden durch Fluor, Chlor oder Brom substituiert sein kann,

X - für Halogen steht,

und

Y - für ein Stickstoffatom oder für die CH-Gruppe steht

und deren Säureadditionssalze.

**3.** Azolylcyclopropyl-azolylmethyl-carbinole gemäß Anspruch 1 in welchen

$R^2$ - für Phenyl oder Naphthyl steht, das gegebenenfalls bis zu 2-fach gleich oder verschieden substituiert ist durch Fluor, Chlor, Brom, $C_1$-$C_4$-Alkyl, $C_1$-$C_4$-Alkoxy, $C_1$-$C_4$-Alkylthio oder durch Halogenalkyl, Halogenalkoxy oder Halogenalkylthio mit bis zu 4 Kohlenstoffatomen im Alkylteil, wobei die Halogenatome gleich oder verschieden sein können, oder durch Phenyl, Phenoxy oder Phenylthio, das seinerseits durch Fluor, Chlor oder Brom substituiert sein kann,

X - für Fluor, Chlor oder Brom steht

und

Y - für ein Stickstoffatom oder für die CH-Gruppe steht,

und deren Säureadditionssalze.

**4.** Verfahren zur Herstellung von Azolyl-cyclopropyl-azolylmethyl-carbinolen der Formel (I) gemäß Anspruch 1 sowie von deren Säureadditionssalzen, dadurch gekennzeichnet, daß man halogenierte Azolylcyclopropylketone der Formel

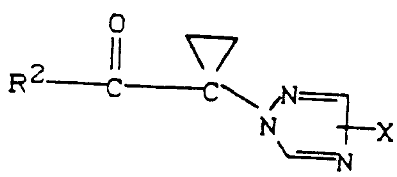

(II)

in welcher

$R^2$ und X die in Anspruch 1 angegebene Bedeutung haben,

zunächst mit Dimethylsulfoniummethylid oder Dimethyloxosulfoniummethylid der Formel

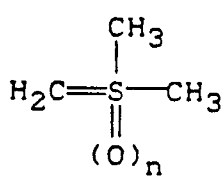

(III)

worin

n - eine Zahl 0 oder 1 bedeutet,

in Gegenwart eines Verdünnungsmittels zu Verbindungen der Formel

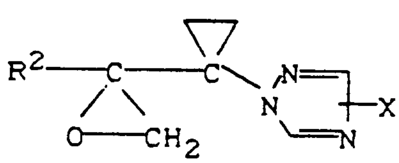

(IV)

in welcher

$R^2$ und X die in Anspruch 1 angegebene Bedeutung haben,

umsetzt
und anschließend
mit Azolen der Formel

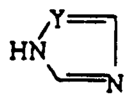

(V)

in welcher
Y die in Anspruch 1 angegebene Bedeutung hat,
in Gegenwart eines inerten organischen Verdünnungsmittels und in Gegenwart einer Base umsetzt und an die so erhaltenen Stoffe der Formel (I) gegebenenfalls anschließend eine Säure addiert.

**5.** Verfahren zur Herstellung von Azolyl-cyclopropyl-azolylmethyl-carbinolen der Formel (I) gemäß Anspruch 1 sowie von deren Säureadditionssalzen, dadurch gekennzeichnet, daß man
Diazolyl-Keton-Derivate der Formel

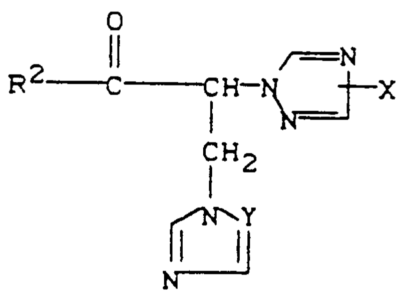

(VII)

in welcher
$R^2$, X und Y die in Anspruch 1 angegebene Bedeutung haben,
zunächst mit mindestens der dreifachen äquivalenten Menge Dimethyloxosulfoniummethylid der Formel

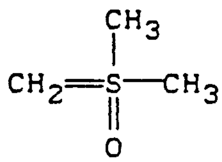

(IIIa)

in Gegenwart eines Verdünnungsmittels zu Verbindungen der Formel

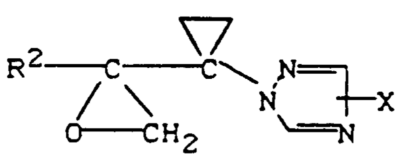

(IV)

in welcher
$R^2$ und X die in Anspruch 1 angegebene Bedeutung haben,
umsetzt
und anschließend
mit Azolen der Formel

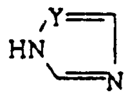

(V)

in welcher
Y die in Anspruch 1 angegebene Bedeutung hat,
in Gegenwart eines inerten Verdünnungsmittels und in Gegenwart einer Base umsetzt und an die so erhaltenen Stoffe der Formel (I) gegebenenfalls anschließend eine Säure addiert.

**6.** Azolylcyclopropylketone der Formel

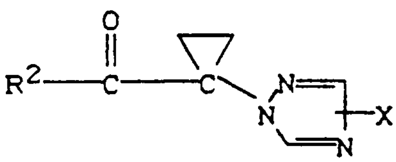

(II)

in welcher
$R^2$ und X die in Anspruch 1 angegebene Bedeutung haben.

**7.** Azolylcyclopropylepoxide der Formel

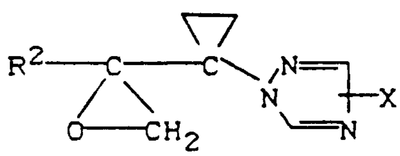

(IV)

in welcher
$R^2$ und X die in Anspruch 1 angegebene Bedeutung haben.

**8.** Diazolylketone der Formel

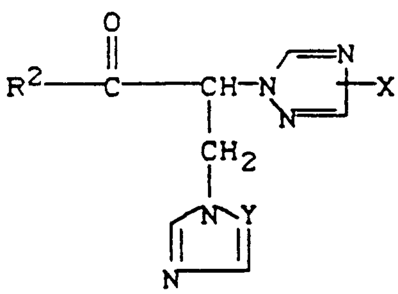

(VII)

in welcher
$R^2$, X und Y die in Anspruch 1 angegebene Bedeutung haben.

**9.** Arzneimittel, gekennzeichnet durch einen Gehalt an mindestens einem Azolylcyclopropyl-azolylmethyl-carbinol der Formel (I) gemäß Anspruch 1 bzw. an einem Säureadditionssalz eines Azolylcyclopropyl-azolylmethyl-carbinols der Formel (I).

**10.** Antimykotische Mittel, gekennzeichnet durch einen Gehalt an mindestens einem Azolylcyclopropyl-azolylmethyl-carbinol der Formel (I) gemäß Anspruch 1 bzw. an einem Säureadditionssalz eines Azolylcyclopropyl-azolylmethyl-carbinols der Formel (I).

**11.** Verwendung von Azolylcyclopropyl-azolylmethyl-carbinolen der Formel (I) gemäß Anspruch 1 bzw. von deren Säureadditionssalzen zur Herstellung von Arzneimitteln.

**12.** Verwendung von Azolylcyclopropyl-azolylmethyl-carbinolen der Formel (I) gemäß Anspruch 1 bzw. von deren Säureadditionssalzen zur Herstellung von antimykotischen Mitteln.

**Claims**

**1.** Azolyl-cyclopropyl-azolylmethyl-carbinols of the formula

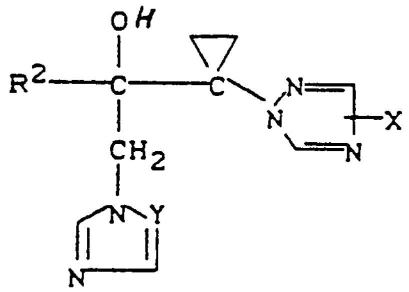

(I)

in which

$R^2$ - represents aryl having 6 to 10 carbon atoms which is optionally monosubstituted to tetrasubstituted by identical or different substituents from the series comprising halogen, $C_1$-$C_8$-alkyl, $C_1$-$C_8$-alkoxy, $C_1$-$C_8$-alkylthio, or by halogenoalkyl, halogenoalkoxy or halogenoalkylthio having up to 8 carbon atoms in the alkyl moiety, where the halogen atoms are identical or different, or by phenyl, phenylthio or phenoxy which, in turn, may be monosubstituted to trisubstituted by identical or different halogen substituents,

X - represents halogen,

and

Y - represents a nitrogen atom or the CH group,

and their acid addition salts.

**2.** Azolyl-cyclopropyl-azolylmethyl-carbinols according to Claim 1 in which

$R^2$ - represents phenyl or naphthyl which is optionally monosubstituted to trisubstituted by identical or different substituents from the series comprising halogen, $C_1$-$C_6$-alkyl, $C_1$-$C_6$-alkoxy, $C_1$-$C_6$-alkylthio or by halogenoalkyl, halogenoalkoxy or halogenoalkylthio having up to 6 carbon atoms in the alkyl moiety, where the halogen atoms are identical or different, or by phenyl, phenylthio or phenoxy which, in turn, may be monosubstituted or disubstituted by identical or different substituents from the series comprising fluorine, chlorine or bromine,

X - represents halogen,

and

Y - represents a nitrogen atom or the CH group,

and their acid addition salts.

**3.** Azolylcyclopropyl-azolylmethyl-carbinols according to Claim 1 in which

$R^2$ - represents phenyl or naphthyl which is optionally monosubstituted or disubstituted by identical or different substituents from the series comprising fluorine, chlorine, bromine, $C_1$-$C_4$-alkyl, $C_1$-$C_4$-alkoxy, $C_1$-$C_4$-alkylthio or by halogenoalkyl, halogenoalkoxy or halogenoalkylthio having up to 4 carbon atoms in the alkyl moiety, where the halogen atoms may be identical or different, or by phenyl, phenoxy or phenylthio which, in turn, may be substituted by fluorine, chlorine or bromine,

X - represents fluorine, chlorine or bromine

and

Y - represents a nitrogen atom or the CH group,

and their acid addition salts.

**4.** Process for the preparation of azolyl-cyclopropyl-azolylmethyl-carbinols of the formula (I) according to Claim 1 and of their acid addition salts, characterized in that halogenated azolylcyclopropyl ketones of the formula

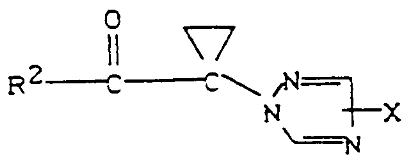

(II)

in which

$R^2$ and X have the meaning given in Claim 1,

are first reacted with dimethylsulphonium methylide or dimethyloxosulphonium methylide of the formula

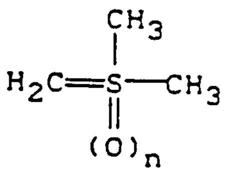

(III)

in which

n - denotes a number 0 or 1,

in the presence of a diluent to give compounds of the formula

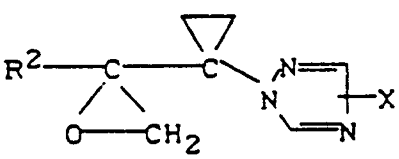

(IV)

in which

$R^2$ and X have the meaning indicated in Claim 1,

and then with azoles of the formula

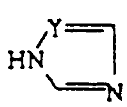

(V)

in which

Y has the meaning indicated in Claim 1,

in the presence of an inert organic diluent and in the presence of a base and, if appropriate, an acid is then added to the substances of the formula (I) thus obtained.

**5.** Process for the preparation of azolyl-cyclopropyl-azolylmethyl-carbinols of the formula (I) according to Claim 1 and of their acid addition salts, characterized in that diazolyl ketone derivatives of the formula

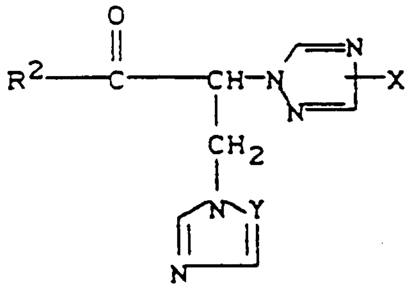

(VII)

in which

$R^2$, X and Y have the meaning given in Claim 1,

are first reacted with at least a three-fold equivalent amount of dimethyloxosulphonium methylide of the formula

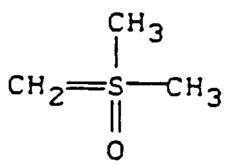

(IIIa)

in the presence of a diluent to give compounds of the formula

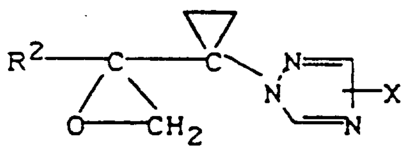

(IV)

in which

$R^2$ and X have the meaning indicated in Claim 1,

and then

with azoles of the formula

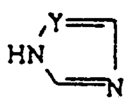

(V)

in which

Y has the meaning given in Claim 1,

in the presence of an inert diluent and in the presence of a base and, if appropriate, an acid is then added to the substances of the formula (I) thus obtained.

**6.** Azolylcyclopropyl ketones of the formula

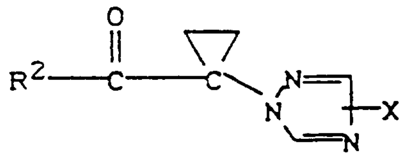

(II)

in which
$R^2$ and X have the meaning indicated in Claim 1.

**7.** Azolylcyclopropyl epoxides of the formula

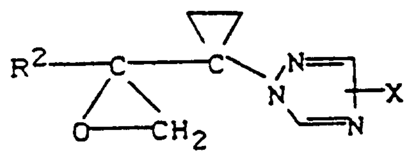

(IV)

in which
$R^2$ and X have the meaning indicated in Claim 1.

**8.** Diazolyl ketones of the formula

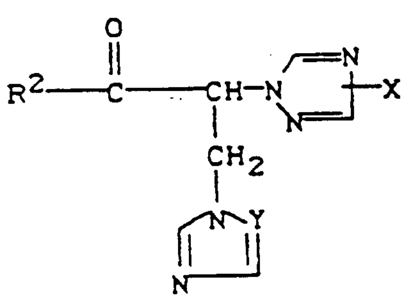

(VII)

in which
$R^2$, X and Y have the meaning indicated in Claim 1.

**9.** Medicaments, characterized in that they contain at least one azolylcyclopropyl-azolylmethyl-carbinol of the formula (I) according to Claim 1 or an acid addition salt of an azolylcyclopropyl-azolylmethyl-carbinol of the formula (I).

**10.** Antimycotic agents, characterized in that they contain at least one azolylcyclopropyl-azolylmethyl-carbinol of the formula (I) according to Claim 1 or an acid addition salt of an azolylcyclopropyl-azolylmethyl-carbinol of the formula (I).

**11.** Use of azolylcyclopropyl-azolylmethyl-carbinols of the formula (I) according to Claim 1 or of their acid addition salts for the production of medicaments.

**12.** Use of azolylcyclopropyl-azolylmethyl-carbinols of the formula (I) according to Claim 1 or of their acid addition salts for the production of antimycotic agents.

## Revendications

**1.** Azolyl-cyclopropyl-azolylméthyl-carbinols de formule

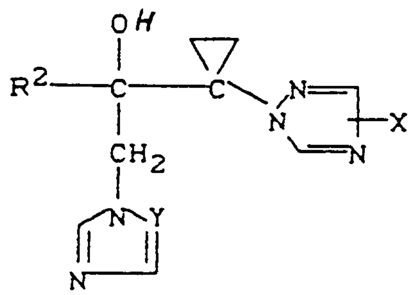

(I)

dans laquelle

R$^2$ - est un groupe aryle ayant 6 à 10 atomes de carbone, qui porte le cas échéant jusqu'à 4 substituants, identiques ou différents, halogéno, alkyle en $C_1$ à $C_8$, alkoxy en $C_1$ à $C_8$, alkylthio en $C_1$ à $C_8$, ou halogénalkyle, halogénalkoxy ou halogénalkylthio ayant jusqu'à 8 atomes de carbone dans la partie alkyle, les atomes d'halogènes étant identiques ou différents, ou phényle, phénylthio ou phénoxy, pouvant porter quant à eux jusqu'à 3 substituants halogéno identiques ou différents,

X - est un halogène et

Y - est un atome d'azote ou le groupe CH

et leurs sels d'addition d'acides.

**2.** Azolyl-cyclopropyl-azolylméthyl-carbinols suivant la revendication 1, dans lesquels

R$^2$ - est un groupe phényle ou naphtyle qui porte le cas échéant jusqu'à 3 substituants, identiques ou différents, halogéno, alkyle en $C_1$ à $C_6$, alkoxy en $C_1$ à $C_6$, alkylthio en $C_1$ à $C_6$, ou bien halogénalkyle, halogénalkoxy ou halogénalkylthio ayant jusqu'à 6 atomes de carbone, les atomes d'halogènes étant identiques ou différents, ou phényle, phénylthio ou phénoxy qui peuvent porter quant à eux jusqu'à 2 substituants fluoro, chloro ou bromo, identiques ou différents,

X - est un halogène

et

Y - est un atome d'azote ou le groupe CH

et leurs sels d'addition d'acides.

**3.** Azolylcyclopropyl-azolylméthyl-carbinols suivant la revendication 1 dans lesquels

R$^2$ - est un groupe phényle ou naphtyle qui porte le cas échéant jusqu'à 2 substituants, identiques ou différents, fluor, chlore, brome, alkyle en $C_1$ à $C_4$, alkoxy en $C_1$ à $C_4$, alkylthio en $C_1$ à $C_4$ ou halogénalkyle, halogénalkoxy ou halogénalkylthio ayant jusqu'à 4 atomes de carbone dans la partie alkyle, les atomes d'halogènes pouvant être identiques ou différents, ou bien phényle, phénoxy ou phénylthio pouvant être substitués quant à eux par du fluor, du chlore ou du brome,

X - est du fluor, du chlore ou du brome

et

Y - est un atome d'azote ou le groupe CH,

et leurs sels d'addition d'acides.

**4.** Procédé de production d'azolyl-cylopropyl-azolylméthyl-carbinols de formule (I) suivant la revendication 1 ainsi que de leurs sels d'addition d'acides, caractérisé en ce qu'on fait réagir des azolylcyclopropyl-cétones halogénées de formule

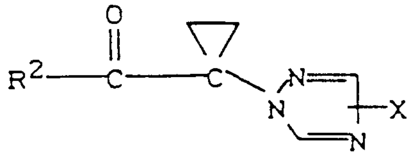

(II)

dans laquelle

$R^2$ et X ont la définition indiquée dans la revendication 1,

tout d'abord avec le méthylure de diméthylsulfonium ou le méthylure de diméthyloxosulfonium de formule

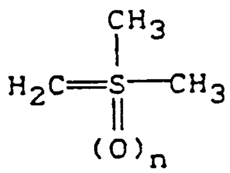

(III)

dans laquelle

n - représente le nombre 0 ou 1,

en présence d'un diluant pour former des composés de formule

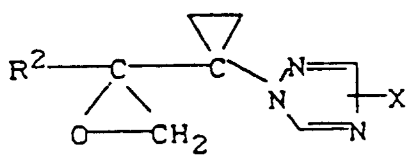

(IV)

dans laquelle

$R^2$ et X ont la définition indiquée dans la revendication 1,

puis

avec des azoles de formule

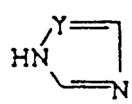

(V)

dans laquelle

Y a la définition donnée dans la revendication 1,

en présence d'un diluant organique inerte et en présence d'une base, puis on additionne éventuellement un acide sur les composés de formule (I) ainsi obtenus.

**5.** Procédé de production d'azolyl-cyclopropyl-azolylméthyl-carbinols de formule (I) suivant la revendication 1 ainsi que de leurs sels d'addition d'acides, caractérisé en ce qu'on fait réagir des dérivés de diazolylcétones de formule

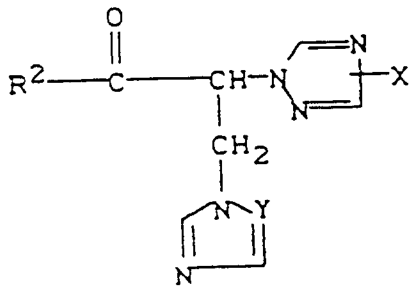

(VII)

dans laquelle

$R^2$, X et Y ont la définition indiquée dans la revendication 1,

tout d'abord avec au moins le triple de la quantité équivalente de méthylure de diméthyloxosulfonium de formule

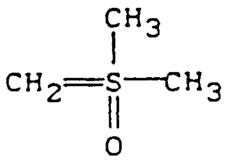

(IIIa)

en présence d'un diluant pour former des composés de formule

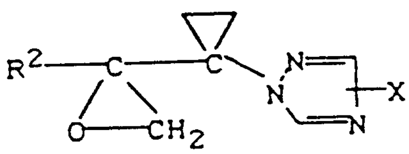

(IV)

dans laquelle

$R^2$ et X ont la définition indiquée dans la revendication 1

que l'on fait réagir ensuite avec des azoles de formule

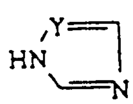

(V)

dans laquelle

Y a la définition indiquée dans la revendication 1,

en présence d'un diluant inerte et en présence d'une base, et on additionne le cas échéant un acide sur les composés de formule (I) ainsi obtenus.

**6.** Azolylcyclopropylcétones de formule

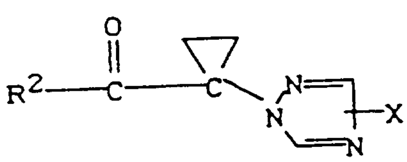

(II)

dans laquelle

$R^2$ et X ont la définition indiquée dans la revendication 1.

**7.** Azolylcyclopropylépoxydes de formule

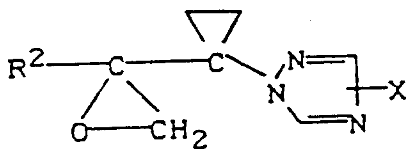

(IV)

dans laquelle

$R^2$ et X ont la définition indiquée dans la revendication 1.

**8.** Diazolylcétones de formule

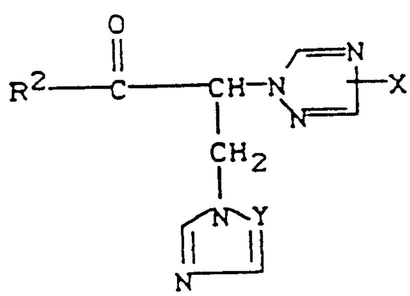

(VII)

dans laquelle

$R^2$, X et Y ont la définition indiquée dans la revendication 1.

**9.** Médicament, caractérisé par une teneur en au moins un azolylcyclopropyl-azolylméthyl-carbinol de formule (I) suivant la revendication 1 ou en un sel d'addition d'acides d'un azolylcyclopropyl-azolylméthyl-carbinols de formule (I).

**10.** Compositions antimycosiques, caractérisées par une teneur en au moins un azolylcyclopropyl-azolylméthyl-carbinols de formule (I) suivant la revendication 1 ou en un sel d'addition d'acides d'un azolylcyclopropyl-azolylméthyl-carbinol de formule (I).

**11.** Utilisation d'azolylcyclopropyl-azolylméthyl-carbinols de formule (I) suivant la revendication 1 ou de leurs sels d'addition d'acides pour la préparation de médicaments.

**12.** Utilisation d'azolylcyclopropyl-azolylméthyl-carbinols de formule (I) suivant la revendication 1 ou de leurs sels d'addition d'acides pour la préparation d'agents antimycosiques.